# EUROPEAN PATENT SPECIFICATION

(11) **EP 1 533 375 B1**
(45) Date of publication and mention of the grant of the patent: **16.09.2009**
(21) Application number: 03764168.5
(22) Date of filing: 11.07.2003
(51) Int. Cl.: C12N 15/09, C12N 15/63, C12N 15/90, C12Q 1/68

(54) **METHOD OF TRANSFERRING MUTATION INTO TARGET NUCLEIC ACID**
VERFAHREN ZUR ÜBERTRAGUNG EINER MUTATION IN EINE ZIELNUKLEINSÄURE
PROCEDE DE TRANSFERT DE MUTATION DANS UN ACIDE NUCLEIQUE CIBLE

(30) Priority: 12.07.2002 JP 2002204887; 18.04.2003 JP 2003113534
(43) Date of publication of application: 25.05.2005
(73) Proprietor: TAKARA BIO INC., Otsu-shi, Shiga 520-2193 (JP)
(72) Inventor: CAO, Chunyu 402, Hamoparesu-Kusatsu, Kusatsu-shi, Shiga 525-0025 (JP); SAGAWA, Hiroaki, Otsu-shi, Shiga 520-2193 (JP); KATO, Ikunoshin, Otsu-chi, Shiga 520-2193 (JP)
(74) Representative: Schnappauf, Georg
(86) International application number: PCT/JP2003/008816
(87) International publication number: WO 2004/007715

(56) References cited:
- WO-A-01/49844
- WO-A2-00/73478
- US-A- 5 643 762
- PICCIN A ET AL: "Efficient and heritable functional knock-out of an adult phenotype in Drosophila using a GAL4-driven hairpin RNA incorporating a heterologous spacer." 15 June 2001 (2001-06-15), NUCLEIC ACIDS RESEARCH. 15 JUN 2001, VOL. 29, NR. 12, PAGE(S) E55 - E55 , XP002334559 ISSN: 1362-4962 * figure 1 *
- WALDMAN ALAN S ET AL: "Long inverted repeats are an at-risk motif for recombination in mammalian cells." GENETICS, vol. 153, no. 4, December 1999 (1999-12), pages 1873-1883, XP002334555 ISSN: 0016-6731
- BZYMEK MALGORZATA ET AL: "Evidence for two mechanisms of palindrome-stimulated deletion in Escherichia coli: Single-strand annealing and replication slipped mispairing" GENETICS, vol. 158, no. 2, June 2001 (2001-06), pages 527-540, XP002334556 ISSN: 0016-6731
- LOBACHEV KIRILL S ET AL: "Factors affecting inverted repeat stimulation of recombination and deletion in Saccharomyces cerevisiae" GENETICS, vol. 148, no. 4, April 1998 (1998-04), pages 1507-1524, XP002334557 ISSN: 0016-6731
- STEINWAERDER D.S. ET AL.: 'Generation of adenovirus vectors devoid of all viral genes by recombination between inverted repeats' J. VIROL. vol. 73, no. 11, 1999, pages 9303 - 9313, XP000914992
- GARCIA-BUSTOS J. ET AL.: 'Nuclear protein localization' BIOCHIM. BIOPHYS. ACTA vol. 1071, no. 1, 1991, pages 83 - 101, XP002113641
- RECCHIA A. ET AL.: 'Site-specific integration mediated by a hybrid adenovirus/adeno-associated virus vector' PROC. NATL. ACAD. SCI. USA vol. 96, no. 6, 1999, pages 2615 - 2620, XP002152917
- ELBASHIR ET AL: "Functional anatomy of siRNAs for mediating efficient RNAi in Drosophila melanogaster embryo lysate" EMBO J, vol. 20, no. 23, 3 December 2001 (2001-12-03), pages 6877-6888,

### SEQUENCE LISTING

<110> TAKARA BIO INC.
<120> Method for introducing mutation into target nucleic acid
<130> 663910
<150> JP 2002-204887
   <151> 2002-07-12
<150> JP 2003-113534
   <151> 2003-04-18
<160> 16
<170> Patent In Ver. 2.1
<210> 1
   <211> 720
   <212> DNA
   <213> Artificial Sequence
<220>
   <223> Description of Artificial Sequence: Gene encoding red-shifted green fluorescence protein.
<400> 1
   atggctagca aaggagaaga actcttcact ggagttgtcc caattcttgt tgaattagat 60
   ggtgatgtta acggccacaa gttctctgtc agtggagagg gtgaaggtga tgcaacatac 120
   ggaaaactta ccctgaagtt catctgcact actggcaaac tgcctgttcc atggccaaca 180
   ctagtcacta ctctgtgcta tggtgttcaa tgcttttcaa gatacccgga tcatatgaaa 240
   cggcatgact ttttcaagag tgccatgccc gaaggttatg tacaggaaag gaccatcttc 300
   ttcaaagatg acggcaacta caagacacgt gctgaagtca agtttgaagg tgataccctt 360
   gttaatagaa tcgagttaaa aggtattgac ttcaaggaag atggaaacat tctgggacac 420
   aaattggaat acaactataa ctcacacaat gtatacatca tggcagacaa acaaaagaat 480
   ggaatcaaag tgaacttcaa gacccgccac aacattgaag atggaagcgt tcaactagca 540
   gaccattatc aacaaaatac tccaattggc gatggccctg tccttttacc agacaaccat 600
   tacctgtcca cacaatctgc cctttcgaaa gatcccaacg aaaagagaga ccacatggtc 660
   cttcttgagt ttgtaacagc tgctgggatt acacatggca tggatgaact gtacaactga 720
<210> 2
   <211> 40
   <212> DNA
   <213> Artificial Sequence
<220>
   <223> Description of Artificial Sequence: PCR primer Us-EcoRI to amplify a gene encoding red-shifted green fluorescence protein.
<400> 2
   cttgaattcg gtaccgagct cggatcgggc gcgcaagaaa 40
<210> 3
   <211> 20
<212> DNA
   <213> Artificial Sequence
<220>
   <223> Description of Artificial Sequence: PCR primer DEND to amplify a gene encoding red-shifted green fluorescence protein. <400> 3
   cactggcggc cgttactagt 20
<210> 4
   <211> 40
   <212> DNA
   <213> Artificial Sequence
<220>
   <223> Description of Artificial Sequence: PCR primer Us-HindIII to amplify a gene encoding red-shifted green fluorescence protein.
<400> 4
   cttaagcttg gtaccgagct cggatcgggc gcgcaagaaa 40
<210> 5
   <211> 40
   <212> DNA
   <213> Artificial Sequence
<220>
   <223> Description of Artificial Sequence: PCR primer U100HindIII to amplify a portion of gene encoding red-shifted green fluorescence protein. <400> 5
   ctaagcttct ggcaaactgc ctgttccatg gccaacacta 40
<210> 6
   <211> 40
   <212> DNA
   <213> Artificial Sequence
<220>
   <223> Description of Artificial Sequence: PCR primer D100BamHI to amplify a portion of gene encoding red-shifted green fluorescence protein. <400> 6
   tcggatccaa gtcatgccgt ttcatatgat ccgggtatct 40
<210> 7
   <211> 37
   <212> DNA
   <213> Artificial Sequence
<220>
   <223> Description of Artificial Sequence: PCR primer Us-EcoRI-1 to amplify a gene encoding red-shifted green fluorescence protein.
<400> 7
   gaattcggta ccgagctcgg atcgggcgcg caagaaa 37
<210> 8
   <211> 37
   <212> DNA
   <213> Artificial Sequence
<220>
   <223> Description of Artificial Sequence: PCR primer Us-HindIII-1 to amplify a gene encoding red-shifted green fluorescence protein.
<400> 8
   aagcttggta ccgagctcgg atcgggcgcg caagaaa 37
<210> 9
   <211> 38
   <212> DNA
   <213> Artificial Sequence
<220>
   <223> Description of Artificial Sequence: PCR primer U100HindIII-1 to amplify a portion of gene encoding red-shifted green fluorescence protein.
<400> 9
   aagcttctgg caaactgcct gttccatggc caacacta 38
<210> 10
   <211> 54
   <212> DNA
   <213> Artificial Sequence
<220>
   <221> modified_base
   <222> (1).. (4)
   <223> um
<220> <221> modified_base
   <222> (50).. (53)
   <223> um
<220>
   <223> Description of Artificial Sequence: Chimeric oligonucleotide ss Oligo.
<400> 10
   uuuuatcttg aaaagcattg aacaccatag cacagagtag tgactagtgu uuut 54
<210> 11
   <211> 35
   <212> DNA
   <213> Artificial Sequence
<223> Description of Artificial Sequence: PCR primer RNA-ecoRI to amplify a portion of gene encoding red-shifted green fluorescence protein."nucleotides 1 to 6 are 2'-0-methyl ribonucleotides - other nucleotides are deoxyribonucleotides"
<400> 11
   gaauucggta ccgagctcgg atcgggcgcg caaga 35
<210> 12
   <211> 35
   <212> DNA
   <213> Artificial Sequence
<220>
   <223> Description of Artificial Sequence: PCR primer RNA-hindIII to amplify a portion of gene encoding red-shifted green fluorescence protein. "nucleotides 1 to 6 are 2'-0-methyl ribonucleotides - other nucleotides are deoxyribonucleotides"
<400> 12
   aagcuuggta ccgagctcgg atcgggagag caaga 35
<210> 13
   <211> 30
   <212> DNA
   <213> homo sapience
<400> 13
   gattgcttta gcttggaaat tccggagctg 30
<210> 14
   <211> 40
   <212> DNA
   <213> Artificial Sequence
<220>
   <223> Description of Artificial Sequence: PCR primer GFP-κB1 to amplify a portion of gene encoding red-shifted green fluorescence protein.
<400> 14
   agctaaagca atctcagttg tacagttcat ccatgccatg 40
<210> 15
   <211> 40
   <212> DNA
   <213> Artificial Sequence
<223> Description of Artificial Sequence: PCR primer GFP-κB2 to amplify a portion of gene encoding red-shifted green fluorescence protein.
<400> 15
   tccggaattt ccaagctaaa gcaatctcag ttgtacagtt 40
<210> 16
   <211> 40
   <212> DNA
   <213> Artificial Sequence
<223> Description of Artificial Sequence: PCR primer GFP-κB3 to amplify a portion of gene encoding red-shifted green fluorescence protein.
<400> 16
   ttttggatcc cagctccgga atttccaagc taaagcaatc 40

### SEQUENCE LISTING

<110> TAKARA BIO INC.
<120> Method for introducing mutation into target nucleic acid
<130> 38-83
<150> JP 2002-204887
   <151> 2002-07-12
<150> JP 2003-113534
   <151> 2003-04-18
<160> 16
<170> PatentIn Ver. 2.1
<210> 1
   <211> 720
   <212> DNA
   <213> Artificial Sequence
<220>
   <223> Description of Artificial Sequence: Gene encoding red-shifted green fluorescence protein.
<400> 1
   atggctagca aaggagaaga actcttcact ggagttgtcc caattcttgt tgaattagat 60
   ggtgatgtta acggccacaa gttctctgtc agtggagagg gtgaaggtga tgcaacatac 120
   ggaaaactta ccctgaagtt catctgcact actggcaaac tgcctgttcc atggccaaca 180
   ctagtcacta ctctgtgcta tggtgttcaa tgcttttcaa gatacccgga tcatatgaaa 240
   cggcatgact ttttcaagag tgccatgccc gaaggttatg tacaggaaag gaccatcttc 300
   ttcaaagatg acggcaacta caagacacgt gctgaagtca agtttgaagg tgataccctt 360
   gttaatagaa tcgagttaaa aggtattgac ttcaaggaag atggaaacat tctgggacac 420
   aaattggaat acaactataa ctcacacaat gtatacatca tggcagacaa acaaaagaat 480
   ggaatcaaag tgaacttcaa gacccgccac aacattgaag atggaagcgt tcaactagca 540
   gaccattatc aacaaaatac tccaattggc gatggccctg tccttttacc agacaaccat 600
   tacctgtcca cacaatctgc cctttcgaaa gatcccaacg aaaagagaga ccacatggtc 660
   cttcttgagt ttgtaacagc tgctgggatt acacatggca tggatgaact gtacaactga 720
<210> 2
   <211> 40
   <212> DNA
   <213> Artificial Sequence
<220>
   <223> Description of Artificial Sequence: PCR primer Us-EcoRI to amplify a gene encoding red-shifted green fluorescence protein.
<400> 2
   cttgaattcg gtaccgagct cggatcgggc gcgcaagaaa 40
<210> 3
   <211> 20
   <212> DNA
   <213> Artificial Sequence
<220>
   <223> Description of Artificial Sequence: PCR primer DEND to amplify a gene encoding red-shifted green fluorescence protein.
<400> 3
   cactggcggc cgttactagt 20
<210> 4
   <211> 40
   <212> DNA
   <213> Artificial Sequence
<220>
   <223> Description of Artificial Sequence: PCR primer Us-HindIII to amplify a gene encoding red-shifted green fluorescence protein.
<400> 4
   cttaagcttg gtaccgagct cggatcgggc gcgcaagaaa 40
<210> 5
   <211> 40
   <212> DNA
   <213> Artificial Sequence
<220>
   <223> Description of Artificial Sequence: PCR primer U100HindIII to amplify a portion of gene encoding red-shifted green fluorescence protein.
<400> 5
   ctaagcttct ggcaaactgc ctgttccatg gccaacacta 40
<210> 6
   <211> 40
   <212> DNA
   <213> Artificial Sequence
<220>
   <223> Description of Artificial Sequence: PCR primer D100BamHI to amplify a portion of gene encoding red-shifted green fluorescence protein.
<400> 6
   tcggatccaa gtcatgccgt ttcatatgat ccgggtatct 40
<210> 7
   <211> 37
   <212> DNA
   <213> Artificial Sequence
<220>
   <223> Description of Artificial Sequence: PCR primer Us-EcoRI-1 to amplify a gene encoding red-shifted green fluorescence protein.
<400> 7
   gaattcggta ccgagctcgg atcgggcgcg caagaaa 37
<210> 8
   <211> 37
   <212> DNA
   <213> Artificial Sequence
<220>
   <223> Description of Artificial Sequence: PCR primer Us-HindIII-1 to amplify a gene encoding red-shifted green fluorescence protein.
<400> 8
   aagcttggta ccgagctcgg atcgggcgcg caagaaa 37
<210> 9
   <211> 38
   <212> DNA
   <213> Artificial Sequence
<220>
   <223> Description of Artificial Sequence: PCR primer U100HindIII-1 to amplify a portion of gene encoding red-shifted green fluorescence protein.
<400> 9
   aagcttctgg caaactgcct gttccatggc caacacta 38
<210> 10
   <211> 54
   <212> DNA
   <213> Artificial Sequence
<220>
   <221> modified_base
   <222> (1)..(4)
   <223> um
<220> <221> modified_base
   <222> (50) .. (53)
   <223> um
<220>
   <223> Description of Artificial Sequence: Chimeric oligonucleotide ss Oligo.
<400> 10
   uuuuatcttg aaaagcattg aacaccatag cacagagtag tgactagtgu uuut 54
<210> 11
   <211> 35
   <212> DNA
   <213> Artificial Sequence
<223> Description of Artificial Sequence: PCR primer RNA-ecoRI to amplify a portion of gene encoding red-shifted green fluorescence protein."nucleotides 1 to 6 are 2'-O-methyl ribonucleotides - other nucleotides are deoxyribonucleotides"
<400> 11
   gaauucggta ccgagctcgg atcgggcgcg caaga 35
<210> 12
   <211> 35
   <212> DNA
   <213> Artificial Sequence
<220>
   <223> Description of Artificial Sequence: PCR primer RNA-hindIII to amplify a portion of gene encoding red-shifted green fluorescence protein. "nucleotides 1 to 6 are 2'-O-methyl ribonucleotides - other nucleotides are deoxyribonucleotides"
<400> 12
   aagcuuggta ccgagctcgg atcgggagag caaga 35
<210> 13
   <211> 30
   <212> DNA
   <213> homo sapience
<400> 13
   gattgcttta gcttggaaat tccggagctg 30
<210> 14
   <211> 40
   <212> DNA
   <213> Artificial Sequence
<220>
   <223> Description of Artificial Sequence: PCR primer GFP-κB1 to amplify a portion of gene encoding red-shifted green fluorescence protein.
<400> 14
   agctaaagca atctcagttg tacagttcat ccatgccatg 40
<210> 15
   <211> 40
   <212> DNA
   <213> Artificial Sequence
<223> Description of Artificial Sequence: PCR primer GFP-κB2 to amplify a portion of gene encoding red-shifted green fluorescence protein.
<400> 15
   tccggaattt ccaagctaaa gcaatctcag ttgtacagtt 40
   <210> 16
   <211> 40
   <212> DNA
   <213> Artificial Sequence
<223> Description of Artificial Sequence: PCR primer GFP-κB3 to amplify a portion of gene encoding red-shifted green fluorescence protein.
<400> 16
   ttttggatcc cagctccgga atttccaagc taaagcaatc 40

## Description

### Technical Field

The present invention relates to a method for introducing a mutation into a target nucleic acid, which is useful four introduction of a mutation into a gene and repair of a mutation in a gene.

### Background Art

A virus vector or the like is used to transfer a gene into a cell that is functionally abnormal due to deletion or mutation of the gene in the cell according to a method currently used for gene therapy. Thereby, the normal function of the cell is restored, and the cell can play its intrinsic role. It may be called gene substitution therapy.

The gene transfer using a virus vector has problems as follows. A mutant gene remains on the chromosome as it is. If a mutant protein derived from the mutant gene has a structure similar to that of a normal protein derived from the normal gene, the mutant protein may interfere with the function of the normal protein.

Recently, attention is paid to gene targeting methods in which a nucleotide in a gene of interest in a cell is converted unlike the virus vector-mediated DNA transfer. The chimera formation method developed by Eric B. Kmiec at Thomas Jefferson University (Proc. Natl. Acad. Sci. USA, Vol.93, p.2071-2076 (1996)) is a specific example of the gene targeting methods. This method utilizes a chimeric oligonucleotide in which a portion containing the nucleotide to be changed is composed of DNA, and RNA stretches are placed at both ends for localization. RNA-DNA binding is stronger than DNA-DNA binding. Thus, if such a chimeric oligonucleotide is transferred into a cell, the RNA nucleotide sequences at both ends search for corresponding nucleotide sequences in the cellular DNA to form a duplex. The mutation is then introduced at the desired site as a result of mismatch repair. The chimeric oligonucleotide has a complicated circular structure. Specifically, maintenance of the structure requires use of extra eight thymine residues, which have considerable negative effects on the ability of binding to a target DNA.

O. Igoucheva et al. has developed the single stranded oligonucleotide (hereinafter also referred to as ss oligo) formation method (Gene Therapy, Vol.8, p.391-399 (2001)). An oligonucleotide of several tens of nucleotides is used according to this method. A nucleotide corresponding to a nucleotide in a target nucleic acid to be mutated is placed at the center of the oligonucleotide, and several methylated uracil residues which are insusceptible to degradation with intracellular nucleases are attached to both ends.

The repair efficiencies of the above-mentioned two mutagenesis methods are still low. The single stranded oligonucleotide (ss oligo) formation method, which results in better repair efficiency among the two, still has a clear drawback. Specifically, the method cannot be used to simultaneously repair mutant nucleotides on both of sense and antisense strands of a double-stranded DNA upon mismatch repair following binding to a target nucleic acid. Only one of the mutant nucleotides is repaired, while the remaining mutant nucleotide on the complementary strand needs to be additionally repaired by means of the mismatch repair mechanism of the cell.

A sequence that has no relationship to a target DNA is included in a chimeric oligonucleotide (chimeric oligo) according to Kmiec et al. for maintaining its circular structure. The unrelated sequence accounts for more than ten percent of the sequence of the oligo. This decreases the activity of targeting a target DNA of the chimeric oligo.

Another important point is that it is impossible to simultaneously repair two or more nucleotides located at a distance in view of the mechanism of repair using a chimeric oligo or ss oligo and the structure of the oligonucleotide to be used. If two or more mutant nucleotides located at a distance are to be repaired, or mutations are to be introduced at two or more sites located at a distance, one has to carry out several rounds of oligonucleotide transfer followed by cloning. The above procedure naturally requires a lot of time and labor. Moreover, current techniques for transferring a DNA into a cell greatly damage the cell. Thus, it is difficult to obtain a viable cell retaining the desired function.

If a mutant nucleotide on a chromosome is to be repaired in a cell, a DNA for repair is only transferred into cytoplasm. The DNA for repair moves to nucleus as a result of free diffusion according to concentration gradient. One has to transfer a large number of the repair DNA molecules into the cell in this case. However, only a few molecules finally enter into nucleus, resulting in unsatisfactory efficiency of mutation repair.

With the development of gene therapy, a highly effective method that enables simultaneous repair of a plurality of nucleotides and active transport of a DNA for repair into nucleus has been desired as a substitute for the above-mentioned less effective repair methods based on complicated mechanisms.

### Summary of Invention

As a result of intensive studies for achieving the above-mentioned objects, the present inventors have found that a nucleotide in a target nucleic acid can be efficiently mutated by using a DNA having an inverted repeat. Thus, the present invention has been completed.

The first aspect of the present invention relates to a method for introducing a mutation into a nucleotide sequence of a target nucleic acid, the method comprising the steps of:
(1) preparing a DNA having an inverted repeat sequence, wherein the nucleotide sequence of the DNA having an inverted repeat sequence is homologous to a target nucleic acid and contains a mutation to be introduced into the target nucleic acid; and
(2) transferring the DNA having an inverted repeat sequence into a cell.

According to the first aspect, the DNA having an inverted repeat sequence may have a binding motif sequence for a protein having a nuclear transport signal such as a binding motif sequence for a transcription factor. The DNA may contain an appropriate modified nucleotide.

According to the first aspect, the target nucleic acid may be a nucleic acid located in cytoplasm or a nucleic acid located in nucleus. The DNA having an inverted repeat sequence may be either a double-stranded DNA or a single-stranded DNA.

According to the method of the first aspect, a plurality of mutations may be simultaneously introduced into the target nucleic acid. The mutation to be introduced into the target nucleic acid is exemplified by substitution, deletion and/or insertion of a nucleotide.

The second aspect of the present invention relates to a kit for introducing a mutation into a target nucleic acid by the method of the first aspect, the kit containing a DNA having an inverted repeat sequence, wherein the nucleotide sequence of the DNA having an inverted repeat sequence is homologous to a target nucleic acid and contains a mutation to be introduced into the target nucleic acid.

The DNA having an inverted repeat sequence contained in the kit of the second aspect may have a binding motif sequence for a transcription factor or an appropriate modified nucleotide. The DNA having an inverted repeat sequence may be either a double-stranded DNA or a single-stranded DNA.

### Brief Description of Drawings

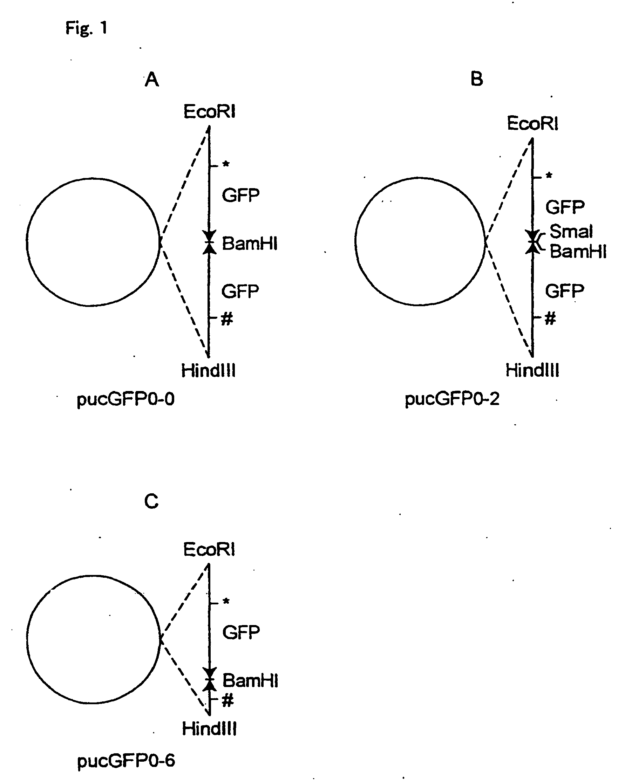

Figure 1 is a schematic drawing of plasmids each having an inverted repeat sequence prepared according to the present invention.

### Detailed Description of the Invention

There is no specific limitation concerning the "target nucleic acid" according to the present invention. Any nucleic acid for which introduction of a mutation is desired may be included. For example, the present invention can be used to introduce a mutation into an intracellular DNA. A DNA located in cytoplasm (an episomal DNA, a plasmid, a mitochondrial DNA, etc.) or a DNA located in nucleus (a chromosomal DNA) can serve as a target nucleic acid.

There is also no specific limitation concerning the mutation to be introduced into a target nucleic acid according to the present invention. It is possible to introduce a mutation such as base substitution, deletion or insertion. In this case, a nucleotide sequence having such a mutation may be included in a DNA having an inverted repeat sequence to be used.

Naturally, introduction of a mutation according to the present invention is not limited only to introduction of a mutation into a normal nucleotide sequence but encompasses an embodiment in which a nucleotide sequence having a naturally occurring mutation is restored to a normal sequence.

The DNA having an inverted repeat sequence used according to the present invention (hereinafter also referred to as an inverted repeat DNA) is a DNA having a nucleotide sequence that is homologous to a target nucleic acid and contains a mutation to be introduced into the target nucleic acid. As used wherein, "homologous" refers to a nucleotide sequence capable of forming a duplex with a target nucleic acid or a strand complementary thereto, and does not mean that a nucleotide sequence is completely matched. That is, it means a nucleotide sequence sufficient for formation of a duplex with a target nucleic acid through base pairing.

There is no specific limitation concerning the method of preparing a DNA having an inverted repeat sequence. Chemical synthesis, enzymatic synthesis (a nucleic acid amplification reaction, etc). or biological synthesis (a method in which a nucleic acid capable of self-replication (e.g., plasmid) is utilized) may be used. A DNA having an inverted repeat sequence is one in which a sense strand sequence and an antisense strand sequence of a target nucleic acid are arranged in tandem. An arbitrary sequence (a spacer) may be inserted between these sequences as long as the resulting DNA can be used to introduce a mutation into the target nucleic acid.

A double-stranded DNA having an inverted repeat sequence can be used according to the present invention. A single-stranded DNA obtained by denaturing the double-stranded DNA may also be used. Since the sense strand portion and the antisense portion of such a single-stranded DNA have nucleotide sequences complementary to each other, the DNA may assume a hairpin structure as a result of base paring of these portions. A single-stranded DNA in this form may also be used according to the present invention.

For example, such a DNA having an inverted repeat sequence and forming a hairpin structure can be prepared according to a method for preparing a single-stranded DNA called slDNA as described in United States Patent Nos. 5,643,762, 5,714,323 and 6,043,028.

It is possible to simultaneously introduce mutations at two or more sites in a target nucleic acid according to the method of the present invention. It is considered to be preferable to make a DNA having an inverted repeat sequence to be used for this purpose as long as possible. DNA homologous recombination repair takes place during a DNA synthesis phase (S phase) in a cell growth cycle. During replication from a parent DNA to a daughter DNA, the helix of the parent DNA is unwound and a replication fork is formed. The targeting activity of a DNA that can simultaneously bind at this time to both the sense and antisense strands of the DNA in the fork portion should be high. Thus, the present inventors prepared a single-stranded DNA that has sequences of sense and antisense strands of a target DNA, i.e., a single-stranded inverted repeat DNA that can bind to both sense and antisense strands of a target DNA.

A plasmid containing an inverted repeat DNA insert in which two identical genes or fragments thereof are arranged in opposite directions is first constructed in the preparation method. The method of constructing a plasmid as described in Example 2 exemplifies such a method. The plasmid can be obtained in large quantities by culturing an *Escherichia coli* cell transformed with the plasmid. An inverted repeat DNA can be obtained by excising the inverted repeat DNA insert from the vector utilizing sites for restriction enzymes at both ends of the insert in the plasmid. Alternatively, the inverted repeat DNA may be amplified by PCR using the plasmid as a template.

Although it is not intended to limit the present invention, it is generally preferably for the construction of the plasmid that a gene or a fragment thereof to be inserted into an inverted repeat DNA is from 500 bp to 1500 bp in length. If the length is less than 500 bp, it may be difficult to insert two identical DNA fragments in opposite directions into a vector plasmid. In this case, the DNA of interest may be synthesized using a different method (e.g., a chemical or enzymatic method).

The following inverted repeat DNA may be used according to the present invention. The DNA has nucleotide sequences of ten nucleotides or more, preferably hundred nucleotides or more, located upstream and downstream of the site in the target nucleic acid at which a mutation is to be introduced. As a result, homologous recombination can readily take place.

If a mutation is to be introduced into a target nucleic acid using a DNA having an inverted repeat sequence according to the present invention (e.g., for correcting a mutant nucleotide in a gene), a mutant nucleotide can be repaired as follows. An inverted repeat DNA having a sequence of a wild type gene which contains a normal nucleotide at the site corresponding to the mutant nucleotide is prepared. It is then transferred into a cell using a known DNA transfer method such as the calcium phosphate method, the electroporation method or the lipid-mediated transfection method.

On the other hand, if a mutation is to be introduced into a wild type gene, the mutation can be introduced into a target gene as follows. An inverted repeat DNA that contains a nucleotide (a nucleotide sequence) to be introduced into the gene is prepared, and then transferred into a cell using the above-mentioned DNA transfer method.

The 5' terminus and the 3' terminus of the DNA having an inverted repeat sequence according to the present invention may be protected from actions of nucleases in order to prevent degradation of the DNA by nucleases. Although it is not intended to limit the present invention, for example, the protection of the termini may be achieved by physically or chemically cyclizing the DNA. Alternatively, a modified nucleotide such as a modified deoxyribonucleotide, a modified ribonucleotide or an LNA (WO 99/14226) may be included in the DNA. Preferably, the termini are protected by incorporating a methylated ribonucleotide, a sulfurized deoxyribonucleotide or the like. Such a nucleotide can be incorporated into terminal portions of a DNA having an inverted repeat sequence by a chemical means, or an enzymatic means as described in Examples below.

The DNA having an inverted repeat sequence used according to the present invention may contain a binding motif sequence for a protein having a nuclear transport signal, i.e., a DNA sequence capable of binding to the protein.

There is no specific limitation concerning the protein having a nuclear transport signal that can be used according to the present invention. A naturally occurring or artificial one may be used. Examples thereof include transcription factors, SV40 large T antigen, histone, nucleoplasmin and a double-stranded RNA-binding protein NF90. In addition, a variety of proteins having nuclear transport signals as described in Biochim. Biophys. Acta, Vol.1071, p.83-101 (1991) can be used according to the present invention.

If a protein having a nuclear transport signal is capable of binding to a specific DNA sequence, a binding motif sequence for the protein may be included in the DNA having an inverted repeat sequence according to the present invention.

There is no specific limitation concerning the binding motif sequence that can be used according to the present invention. For example, a sequence capable of binding to a protein having a nuclear transport signal that is expressed in a cell into which a mutation is to be introduced can be used according to the present invention. Even if a protein having a nuclear transport signal does not have a DNA binding site, or is not known to have a binding site, it can be used according to the present invention by preparing a chimeric protein that has an appropriate DNA binding sequence in addition to the nuclear transport signal.

If a protein having a nuclear transport signal is expressed in a cell into which a mutation is to be introduced, a DNA having an inverted repeat sequence that has a binding motif sequence for the protein is transferred into the cell. Then, the transferred DNA binds to the protein and is transported to nucleus, and the mutation of interest can be introduced into the chromosomal DNA. If a protein having a nuclear transport signal is not expressed in a cell into which a mutation is to be introduced, or is an artificially prepared one, the method of the present invention may be carried out by transferring the protein or a gene encoding the protein into the cell. There is no specific limitation concerning the method for transferring the protein or a gene encoding the protein into a cell. One can utilize a known method such as a method in which liposome or the like is used, or a method in which a plasmid vector, a virus vector or the like is used. The protein or a gene encoding the protein may be co-transferred into a cell with a DNA having an inverted repeat sequence that has a binding motif sequence for the protein.

The sequence capable of binding to a protein having a nuclear transport signal that is preferably used according to the present invention is exemplified by a DNA sequence capable of binding to a transcription factor. As used herein, a transcription factor means a factor that influences efficiency of transcription from DNA to RNA by an RNA polymerase. Examples of binding motif sequences for transcription factors that can be used according to the present invention include, but are not limited to, sequences capable of binding to transcription factors such as NF-κB, Sp1, AP1, NF-IL6 (C/EBPβ), AP2, Oct-1, SRF and Ets-1. For example, the binding motif sequences for the transcription factors are described in the following literatures: Eur. J. Biochem, Vol.268, p.1828-1836 (2001); J. Biol. Chem., Vol.263, p.3372-3379 (1998); Cell, Vol.49, p.741-752 (1987); EMBO J., Vol.9, p.1897-1906 (1990); Proc. Natl. Acad. Sci. USA, Vol.88, p.7948-7952 (1991); Genes Dev., Vol.2, p.1582-1599 (1988); Nucleic Acids Res., Vol.20, p.3297-3303 (1992); Genes Dev., Vol.4, p.1451-1453 (1990).

There is no specific limitation concerning the copy number of a binding motif sequence to be included in the DNA having an inverted repeat sequence according to the present invention. A single copy or plural copies of the sequence may be used. There is also no specific limitation concerning the position of the sequence. It may be located in the 5'- or 3'-terminal portion of the DNA. For example, two or more copies of a binding motif sequence may be included in both 5'- and 3'-terminal portions. Preferably, a binding motif sequence is incorporated in the middle of an inverted repeat sequence portion, i.e., between repeat sequences.

A DNA having an inverted repeat sequence that contains a binding motif for a protein having a nuclear transport signal can be actively transported into nucleus. Thus, it is highly effective in introduction of a mutation into a chromosomal DNA or repair of a mutation.

The method of the present invention can be used to knock out a gene. For example, one may interfere with translation into a protein by substitution of one or two nucleotides in an initiation codon "ATG" in this case. Alternatively, production of a full length translation product may be inhibited by introducing a termination codon at an appropriate position in a target gene. Furthermore, knockout of a gene may be achieved by preparing an inverted repeat DNA in which once or two nucleotide(s) is(are) inserted into (or deleted from) a nucleotide sequence of a target gene, and utilizing it to cause a frame shift in a nucleotide sequence of a protein-encoding region in the target gene. For example, it is desirable to mutate a nucleotide in an initiation codon of a gene, a nucleotide within 2-30 nucleotides from an initiation codon, a nucleotide encoding an amino acid residue constituting an active site of an enzyme (in case of an enzyme protein), or a nucleotide encoding an amino acid residue that is determinative for fluorescence (in case of a fluorescence-emitting protein).

The most important feature of the present invention is that it can be used to simultaneously repair two or more mutant nucleotides located at a distance. A plurality of mutations can be repaired in a manner similar to that for repair of a single mutant nucleotide using an inverted repeat DNA. Such a DNA consists of several hundreds to several thousands of nucleotides and contains nucleotides for repairing the plurality of mutant nucleotides in the target nucleic acid within a region of several hundreds to several thousands of nucleotides. For example, two mutant nucleotides located at a distance of about 200 nucleotides in a gene can be repaired at a time as described in Example 5.

In the above-mentioned embodiment where mutations are introduced at two or more sites, there is no specific limitation concerning the distance between the sites. The sites may be separated by several hundreds of nucleotides. In view of mutagenesis efficiency, the sites are located preferably within 200 nucleotides, more preferably within 100 nucleotides, most preferably within 30 nucleotides.

The present invention provides a kit used for introducing a mutation according to the present invention. In one embodiment, the kit contains a DNA having an inverted repeat sequence for introducing a mutation into a target nucleic acid. In addition, the kit may contain a reagent for transferring the DNA into a cell.

Examples of the cells include, but are not limited to, blood cells (hematopoietic cells, hematopoietic stem cells, bone marrow cells, umbilical cord blood cells, peripheral blood stem cells, mononuclear cells, lymphocytes, B cells, T cells, etc.), fibroblasts, neuroblasts, nerve cells, endothelial cells, vascular endothelial cells, hepatic cells, myoblasts, skeletal muscle cells, smooth muscle cells, cancer cells and leukemia cells.

One exemplary application of the present invention is convenient production of a non-human transgenic animal (e.g., mouse, rat, dog, pig, cow, chicken, frog or medaka) using a reproductive cell (e.g., embryonic stem cell, primordial germ cell, oocyte, oogonium, ovum, spermacyte or sperm) as a target cell. For example, a gene knockout animal in which only expression of a gene of interest is specifically suppressed can be produced according to the method of the present invention. Such a knockout animal is very useful for functional analysis of the gene, screening of an agent related to the function of the gene or the like.

### Examples

The following examples further illustrate the present invention in detail but are not to be construed to limit the scope thereof.

### Example 1

### Introduction of mutant nucleotide into red-shift green fluorescent protein gene

A single nucleotide in a nucleotide sequence of a red-shift green fluorescent protein (hereinafter referred to as GFP)-encoding gene (SEQ ID NO:1) inserted in a plasmid pQBI25 (Quantum Biotechnologies Inc.) was subjected to substitution such that the gene was not expressed. Amino acid residues at positions 66 to 68 are important for fluorescence of GFP. A codon "TAT" which encodes tyrosine at position 67 among them was changed to a termination codon "TAG" using PCR in vitro mutagenesis kit (Takara Bio). A mutant GFP (mGFP) gene prepared as described above and a GFP-encoding gene without a mutation were independently inserted into a plasmid pDON-AI (Takara Bio) to construct pDON-mGFP and pDON-GFP, respectively. 50 µl of TE buffer containing 1 µg of one of these plasmids was mixed with 50 µl of Optimen medium (Gibco BRL) containing 1 µg of lipofectamine 2000 (LF2000, Gibco BRL). The mixture was used to transfect 293 cells. The cells were observed under a fluorescence microscope after four days. Intense green fluorescence was observed for cells to which pDON-GFP was added, while no fluorescence was observed for cells containing pDON-mGFP. Based on the results, a mutant gene that does not emit fluorescence in a cell was constructed. The mGFP gene was used in the following experiments.

### Example 2

For preparing an inverted repeat DNA (hereinafter referred to as irDNA) having a nucleotide sequence of the GFP gene, a set of two fragments of the GFP gene in opposite directions as a template for amplification of irDNA was first incorporated into a plasmid. A fragment of the entire sequence of the GFP gene inserted in the plasmid pQBI25 was amplified using primers Us-EcoRI (SEQ ID NO:2) and DEND (SEQ ID NO:3). A 760-bp DNA fragment obtained by digesting the fragment with EcoRI and BamHI (both from Takara Bio) was incorporated between EcoRI and BamHI sites in a plasmid pUC19 (Takara Bio). A DNA fragment of the entire sequence of the GFP gene was obtained by amplification using primers Us-hindIII (SEQ ID NO:4) and DEND. A 760-bp DNA fragment obtained by digesting the DNA fragment with HindIII (Takara Bio) and BamHI was incorporated between HindIII and BamHI sites in this plasmid. The plasmid constructed as described above was designated as pucGFPO-0.

Furthermore, a fragment of the entire sequence of the GFP gene was amplified using primers Us-EcoRI and DEND. A 714-bp DNA fragment obtained by digesting the fragment with EcoRI and PvuII (Takara Bio) was incorporated between EcoRI and SmaI sites in the plasmid pUC19. A DNA fragment of the entire sequence of the GFP gene was obtained by amplification using primers Us-hindIII and DEND. A 760-bp DNA fragment obtained by digesting the DNA fragment with HindIII and BamHI was incorporated between HindIII and BamHI sites in this plasmid. The plasmid constructed as described above was designated as pucGFPO-2.

In addition, a DNA fragment was amplified from the GFP gene using primers U100hindIII (SEQ ID NO:5) and U100bamhI (SEQ ID NO:6). A 110-bp DNA fragment was prepared by digesting the DNA fragment with HindIII and BamHI. The 110-bp DNA fragment was substituted for the 760-bp HindIII-BamHI fragment in the plasmid pucGFPO-0 to construct a plasmid pucGFP0-6.

pucGFP0-0 has an inverted repeat sequence for the full length GFP-encoding gene. The length of the insert DNA is 1518 bp. A region of 38 nucleotides from the termination codon is deleted in one of the repeat sequences in pucGFP0-2. The length of the insert DNA is 1479 bp. The plasmid pucGFP0-6 has an inverted repeat sequence composed of the full length GFP-encoding gene and nucleotides from position 150 to position 250 of the GFP gene. The length of the insert DNA is 868 bp. Figures 1A, 1B and 1C illustrate pucGFP0-0, pucGFP0-2 and pucGFP0-6, respectively.

Inverted repeat DNAs, 0-0 irDNA, 0-2 irDNA and 0-6 irDNA, were prepared by PCRs using pucGFP0-0, pucGFP0-2 and pucGFP0-6 as templates. Primers Us-ecoRI-1 (SEQ ID NO:7) and Us-hindIII-1 (SEQ ID NO:8) were used for the preparation of 0-0 irDNA and 0-2 irDNA. Primers Us-ecoRI-1 and U100hindIII-1 (SEQ ID NO:9) were used for the preparation of 0-6 irDNA. The nucleotides "T" and "A" of the wild type GFP gene for restoring the mutant nucleotides "G" and "C" introduced into the mGFP gene are located at the 237th and 1282nd nucleotides, the 237th and 1243rd nucleotides, and the 237th and 813th nucleotides from the 5' terminus of the sense strand or the 3' terminus of the antisense strand (the EcoRI site) in 0-0 irDNA, 0-2 irDNA and 0-6 irDNA, respectively. The positions of nucleotides involved in repair of the mutant nucleotides in the sense or antisense strand of the mGFP gene are indicated by marks "*" and "#" in Figure 1.

### Example 3

### Repair of single mutant nucleotide in mGFP gene in episome experimental model

Experiments of gene repair using inverted repeat DNAs were carried out, and binding to a target nucleic acid and repair of a mutant nucleotide in a target nucleic acid were examined. Influence of toxicity to cells due to plural rounds of DNA transfer was avoided by simultaneous transfer into cytoplasm of a plasmid having the incorporated mGFP gene (pcep-mGFP) as a target nucleic acid, and one of the three inverted repeat DNAs (irDNAs) for repair or ss oligo as a control. The nucleotide sequence of ss oligo is shown in SEQ ID NO:10.

### (1) Repair of episomal mGFP gene using heat-denatured irDNA

For preparing an experimental model, a 760-bp HindIII-BamHI fragment containing the mGFP gene was isolated from pDON-mGFP constructed in Example 1, and subcloned between HindIII and BamHI sites in an episomal mammalian expression vector pCEP4 (Invitrogen) to prepare a plasmid pcep-mGFP. 293 cells (8 x 10⁴) were seeded into a 48-well plate and cultured overnight in DEME medium containing 10% FBS. 2 µg each of 0-0 irDNA, 0-2 irDNA and o-6 irDNA was heat-denatured at 94°C for 5 minutes and cooled rapidly in ice water. 2 µg of ss oligo, 0-0 irDNA, 0-2 irDNA or 0-6 irDNA, and 1.5 µg of prep-mGFP or pUC19 (negative control 1) were diluted with 37.5 µl of Optimen medium. The mixture was mixed with the same volume of Optimen medium containing 2 µg of LF2000. A mixture prepared by mixing 37.5 µl of Optimen medium containing only 3.5 µg of pcep-mGFP and the same volume of Optimen medium containing 2 µg of LF2000 was used as negative control 2.

After incubating for 20 minutes, each of the DNA-LF2000 reagent complexes was added to the cells. The mixture was allowed to stand at room temperature for 30 minutes. 150 µl of Optimen medium was further added thereto. After transfection for 6 hours, 1 ml of DEME medium containing 10% FBS was added thereto. After 48 hours, no green fluorescence-emitting cell (GFP-positive cell) was observed for the negative controls 1 and 2. On the other hand, GFP-positive cells were observed for wells in which ss oligo or one of the three irDNAs for repair of mutant nucleotides and pcep-mGFP containing the target DNA were used for transfection. The maximal number of GFP-positive cells was observed on the fourth day. The cells were detached from the plate by trypsinization, and GFP-positive cells were determined using FACS. The results obtained by subtracting the values for the negative control 1 as backgrounds from the determined values are shown in Table 1.

The greatest value for the number of GFP-positive cells per 10⁴ of 293 cells as an index of repair of mutant nucleotides in the mGFP gene was observed in case of 0-0 irDNA. The repair effect was about 3-fold higher than that observed with ss oligo. As a result of Mann-Whitney U test for four rounds of independent experiments, a significant difference (p<0.05) was observed between the repair efficiencies of 0-0 irDNA and ss oligo.

**Table 1**

| DNA | Number of GFP-positive cells per 10⁴ cells |
|---|---|
| 0-0 irDNA | 18.21 ± 4.22 |
| 0-2 irDNA | 11.79 ± 2.84 |
| 0-6 irDNA | 5.26 ± 0.74 |
| ss oligo | 5.40 ± 2.64 |

### (2) Repair of episomal mGFP gene using irDNA without heat denaturation

Repair of the mGFP gene was examined as described Example 3-(1) except that irDNA prepared by PCR amplification were used without heat denaturation. The number of GFP-positive cells appeared in 10⁴ of 293 cells is shown in Table 2. Repair efficiencies higher than that with ss oligo were observed using the respective irDNAs without heat denaturation. As a result of Mann-Whitney U test for four rounds of independent experiments, a significant difference (p<0.05) was observed between the repair efficiencies of 0-0 irDNA or 0-2 irDNA and ss oligo.

**Table 2**

| DNA | Number of GFP-positive cells per 10⁴ cells |
|---|---|
| 0-0 irDNA | 39.27 ± 9.21 |
| 0-2 irDNA | 20.78 ± 2.80 |
| 0-6 irDNA | 13.12 ± 2.15 |
| ss oligo | 6.54 ± 2.64 |

When pcep-mGFP as a target nucleic acid and 0-0 irDNA for mutation repair were used for sequential transfections conducted at an interval of 6 hours, repair efficiency higher than that observed using ss oligo was observed.

It was shown in the experiments as described in (2) above that the plasmid as a target nucleic acid was not transferred into all of the cells used. pcep-GFP and 0-0 irDNA were transferred into 293 cells under the same conditions and the number of GFP-positive cells was determined. As a result, only 21.22 ± 4.67% of the cells were shown to harbor the plasmid. The results shown in Table 2 were converted by eliminating cells without the plasmid. The number of GFP-positive cells in which the mutant nucleotides were repaired per 10⁴ cells having transferred pcep-mGFP is shown in Table 3. As a result of Mann-Whitney U test for four rounds of independent experiments, a significant difference (p<0.05) was observed between the repair efficiencies of 0-0 irDNA and ss oligo.

**Table 3**

| DNA | Number of GFP-positive cells per 10⁴ cells having transferred pcep-mGFP |
|---|---|
| 0-0 irDNA | 201.42 ± 58.42 |
| 0-2 irDNA | 109.12 ± 27.48 |
| 0-6 irDNA | 72.24 ± 23.97 |
| ss oligo | 39.76 ± 20.94 |

The amounts of the three irDNAs and ss oligo used for transfection in the above-mentioned experiments (2 µg) correspond to 529 nmol (ss oligo), 9.5 nmol (0-0 irDNA), 10.1 nmol (0-2 irDNA) and 16.6 nmol (0-6 irDNA). The rates of repairing mutant nucleotides with the three irDNAs were higher than that with ss oligo. Even if transfection was carried out without denaturation, high repair efficiency similar to that observed upon transfection using heat-denatured irDNA was observed using 0-0 irDNA which contains the entire GFP gene region. The repair efficiency was 5-fold higher than that with ss oligo. Although the number of 0-0 irDNA molecules was several tens of times fewer than the number of ss oligo molecules, the repair efficiency with 0-0 irDNA was several times higher than that with ss oligo. The experimental results suggest that the activity of targeting a target nucleic acid of irDNA is considerably higher than that of ss oligo, leading to increased repair efficiency.

### Example 4

### Incorporation of mGFP gene into chromosome of cell

Retrovirus particles for incorporating the mGFP gene into a chromosome of a cell were prepared using Retrovirus packaging kit ampho (Takara Bio). The recombinant retrovirus vector pDON-mGFP as described in Example 1 was co-transferred into 293 cells with a packaging vector in the kit according to the calcium phosphate method. After culturing for 48 hours, a culture supernatant was collected and filtrated. The culture supernatant (retrovirus suspension) was diluted and added to a medium for 293 cells. It was confirmed that a cloned cell (293-10 cell) contained the mGFP gene by sequence analysis of a PCR-amplified DNA fragment. A genomic DNA was extracted from the 293-10 cell and the sequences of the integration site for the retrovirus vector and the cellular chromosomal DNAs adjacent to the site were analyzed. It was then confirmed that a single copy of the GFP gene was incorporated into the cell. This cloned cell was used in the following experiments.

### Example 5

### Preparation of 0-0 irDNA for repair modified with methylated ribonucleotide and repair of mutant nucleotide in mGFP

Nucleotide sequences of a 5' primer RNA-ecoRI and a 3' primer RNA-hindIII which were synthesized using 2'-0-methyl RNA phosphoamidite and CE-phosphoamidite according to the phosphoamidite method are shown in SEQ ID NOS:11 and 12, respectively. The first six nucleotides are methylated ribonucleotides in each primer. Six methylated ribonucleotides are attached at the 5' termini of the sense strand and the antisense strand of 0-0 irDNA obtained by carrying out PCR using these two primers and pcuGFP0-0 as a template. The 0-0 irDNA that is insusceptible to degradation with nucleases in cells was designated as R0-0 irDNA.

4 x 10⁵ of 293-10 cells or 293 cells (for negative control) were seeded into each well of a 24-well plate and cultured overnight in DEME medium containing 10% FBS. 4 µg of ss oligo, 0-0 irDNA or R0-0 irDNA was diluted with 75 µl of Optimen medium. The mixture was mixed with the same volume of Optimen medium containing 4 µg of LF2000. After incubating for 20 minutes, each of the DNA-LF2000 reagent complexes was added directly to the cells. 90 µl of DEME medium containing 10% FBS was further added thereto for transfection. The amounts in moles of ss oligo, 0-0 irDNA and R0-0 irDNA in the medium were 992.00 nmol, 17.83 nmol and 17.82 nmol, respectively. After 6 hours, 1.5 ml of DEME medium containing 10% FBS was added thereto. After 16-18 hours, the medium was exchanged for DEME medium containing 3% FBS. The cultivation was continued at 32°C for two and a half days.

For accurately counting GFP-positive cells, the cells were washed with PBS, detached by trypsinization and transferred to wells of a new well plate containing a medium, and the number of GFP-positive cells in each well (1.6 to 1.9 x 10⁶ cells) was counted under a fluorescence microscope. The background values for GFP-positive cells in 293 cells as controls were subtracted from the values for GFP-positive cells in 293-10 cells to which the DNA for repair had been added. As a result, the average numbers of GFP positive cells per well with ss oligo, 0-0 irDNA and R0-0 irDNA were about 54, 2300 and 3800, respectively. The number of GFP-positive cells per 10⁴ of 293-10 cells is shown in Table 4.

As a result of Mann-Whitney U test for four rounds of independent experiments, a significant difference (p<0.05) was observed between the repair efficiencies of each of the two irDNAs and ss oligo.

**Table 4**

| DNA | Number of GFP-positive cells per 10⁴ cells |
|---|---|
| 0-0 irDNA | 12.32 ± 4.08 |
| R0-0 irDNA | 24.20 ± 3.84 |
| ss oligo | 0.31 ± 0.08 |

As described above, the rate of repairing mutant nucleotides in the chromosome of 293-10 cell with R0-0 irDNA was higher than that with ss oligo or 0-0 irDNA. The experimental results suggest that mutation repair efficiency was increased due to resistance of the methylated ribonucleotide to degradation by nucleases or the like, and prolonged retention of R0-0 irDNA in cytoplasm or nucleus as compared with 0-0 irDNA.

### Example 6

### Repair of mutant nucleotide using irDNA containing sequence capable of binding to transcription factor

Transcription factors are important factors that are involved in signal transduction to nucleus, and play roles in gene expression control. Many of transcription factors are synthesized in cytoplasm and are always waiting their turns in cytoplasm. If a transcription factor receives an activating signal, it is transported into nucleus and functions.

A transcription factor NF-κB and I-κB which suppresses the activity of NF-κB are mostly present in cytoplasm beinq bound to each other. If I-κB is phosphorylated as a result of stimulus by a cytokine such as TNF-α, NF-κB is activated and transported into nucleus (Proc. Natl. Acad. Sci. USA, Vol.91, p.11884-11888 (1994)). NF-κB binds to a short DNA sequence called NF-κB motif located upstream of a gene and promotes transcription of the gene.

The presence of NF-κB in 293 cells and the DNA sequence capable of binding thereto (5'-gattgctttagcttggaaattccggagctg-3', SEQ ID NO:13) have been reported (Eur. J. Biochem., Vol.268, p.1828-1836 (2001)). Three primers GFP-κB1 (SEQ ID NO:14), GFP-κB2 (SEQ ID NO:15) and GFP-κB3 (SEQ ID NO:16) were synthesized in order to introduce this sequence into an inverted repeat sequence in the middle of irDNA. First, a PCR reaction was conducted using GFP-κB1 and the above-mentioned primer Us-EcoRI as well as an EcoRI-BamHI fragment excised from pucGFP 0-0, which contained the GFP gene, as a template DNA to obtain an amplified DNA fragment. Next, PCR was carried out using this amplified fragment as a template as well as GFP-κB2 and Us-EcoRI to obtain an amplified fragment. Furthermore, PCR was carried out using this amplified fragment as a template as well as GFP-κB3 and Us-EcoRI to obtain an amplified fragment. The resulting amplified fragment was digested with EcoRI and BamHI, and the EcoRI-BamHI fragment in pucGFR0-0 was replaced by the fragment resulting from the digestion. The plasmid constructed as described above was designated as pucGFP0nf-0.

pucGFP0nf-0 contains an insert that comprises an inverted repeat sequence for the GFP gene and the NF-κB-binding motif sequence. The length of the insert is 1548 bp. PCR was carried out using this plasmid as a template DNA as well as the primers RNA-ecoRI and RNA-hindIII, or S-ecoRI and S-hindIII. S-ecoRI and S-hindIII are primers whose sequences are identical to the primers RNA-ecoRI and RNA-hindIII, respectively, and in which six nucleotides from the 5' terminus of each primer are changed to deoxyribonucleotides, and the phosphate groups between them are modified with sulfur molecules. The amplified fragments were designated as R0nf-0 irDNA and S0nf-0 irDNA, respectively.

The preculture conditions for 293-10 cells and 293 cells (negative control) and the method for transfection of DNAs for repair were the same as those described in Example 5 except that TNF-α was included at a concentration of 8 ng/ml in the medium added 6 hours after transfer of ss oligo, R0-0 irDNA, R0nf-0 irDNA or S0nf-0 irDNA.

The cells were treated, the number of GFP-positive cells in each well was counted under a fluorescence microscope, and the background values for GFP-positive cells in 293 cells as controls were subtracted from the values for GFP-positive cells in the cells to which the DNA for repair had been added as described in Example 5. As a result, the average numbers of GFP-positive cells with ss oligo, R0-0 irDNA, R0nf-0 irDNA and S0nf-0 irDNA were 88, about 6000, more than 10,000 and more than 10,000, respectively. The number of GFP-positive cells per 10⁴ of 293-10 cells with ss oligo, R0-0 irDNA, R0nf-0 irDNA or S0nf-0 irDNA is shown in Table 5. The highest repair efficiency observed using S0nf-0 irDNA was 138-fold higher than that observed with ss oligo.

As a result of Mann-Whitney U test for four rounds of independent experiments, a significant difference (p<0.05) was observed between the repair efficiencies of each of the three irDNAs and ss oligo. Also, a significant difference (p<0.05) was observed between the repair efficiencies of R0-0 irDNAs and each of R0nf-0 irDNA and S0nf-0 irDNA having the introduced binding motif sequences for transcription factor.

**Table 5**

| DNA | Number of GFP-positive cells per 10⁴ cells |
|---|---|
| R0-0 irDNA | 28.19 ± 6.54 |
| R0nf-0 irDNA | 61.02 ± 13.72 |
| S0nf-0 irDNA | 67.58 ± 20.31 |
| ss oligo | 0.49 ± 0.14 |

ss oligo or 0-0 irDNA transferred into cytoplasm using lipofectamine moves to nucleus in a manner of passive diffusion as a result of free diffusion according to concentration gradient. Since the concentration of 0-0 irDNA was several tens of times lower than that of ss oligo and therefore the number of 0-0 irDNA copies transported into nucleus was fewer, it had been expected that the rate of repairing mutant nucleotides with 0-0 irDNA might become lower than that with ss oligo. However, the actual results showed that the repair rate with 0-0 irDNA was higher than that with ss oligo. These results suggests that a high rate of repairing mutant nucleotides with 0-0 irDNA was achieved due to the high targeting activity of 0-0 irDNA in spite of the few number of copies transported into cellular nucleus. Furthermore, the repair effect is increased by introducing, into irDNA, a short DNA sequence capable of binding to a transcription factor to promote transport of the irDNA into nucleus. The effect is further increased by modifying the irDNA at the 5' terminus with a methylated ribonucleotide or a sulfurized deoxyribonucleotide which is insusceptible to degradation with nucleases.

The repair effect of a DNA having the same sequence as a target DNA without an inverted repeat structure to which the NF-κB-binding motif sequence was attached was several times lower than that of an inverted repeat DNA (0-nf-0 irDNA). The effect of a DNA into which two molecules of the NF-κB-binding motif sequence were introduced in opposite directions in the middle of 0-0 irDNA was about a half of the effect of 0nf-0 irDNA having a single molecule of the NF-κB-binding motif sequence introduced.

### Example 7

### Repair of GFP gene having double mutations

The residue "G" in the initiation codon "ATG" of the GFP gene was changed to "T" by applying the PCR in vitro mutagenesis method. The mutant GFP (m1GFP) gene was subcloned into pCEP4 and used to transfect 293 cells, and it was confirmed that fluorescence was not observed. Furthermore, a HindIII-NheI fragment (-36-174) in the upstream region of the mGFP gene prepared in Example 1 was replaced by a HindIII-NheI fragment (-36-174) of the m1GFP gene to construct a double mutant GFP (dmGFP) gene.

The dmGFP gene has two mutant nucleotides ("T" at position 3 and "G" at position 201) separated by 198 nucleotides. A 760-bp HindIII-BamHI fragment of the dmGFP gene was subcloned between HindIII and BamHI sites in pCEP4 to prepare a plasmid to be used as a target nucleic acid, pcep-dmGFP.

The efficiency of simultaneously repairing the double mutations using 0-0 irDNA was examined under the experimental conditions as described in Example 3. As a result, GFP-positive cells indicative of simultaneous repair of the two mutant nucleotides were observed at a rate of 15.85 ± 2.00 per 10⁴ cells.

### Industrial Applicability

The present invention describes a method that enables introduction of a mutation into a nucleotide sequence of a gene with high efficiency. Introduction of an artificial mutation into a DNA in a cell or repair of a nonfunctional gene due to a mutation is possible according to the description. The method of the present invention is useful for production of knockout organisms, functional analyses of genes and the like.

### Sequence Listing Free Text

SEQ ID NO:1; Gene encoding red-shifted green fluorescence protein.
SEQ ID NO:2; PCR primer Us-EcoRI to amplify a gene encoding red-shifted green fluorescence protein.
SEO ID NO:3; PCR primer DEND to amplify a gene encoding red-shifted green fluorescence protein.
SEQ ID NO:4; PCR primer Us-HindIII to amplify a gene encoding red-shifted green fluorescence protein.
SEQ ID NO:5; PCR primer U100HindIII to amplify a portion of gene encoding red-shifted green fluorescence protein.
SEQ ID NO:6; PCR primer D100BamHI to amplify a portion of gene encoding red-shifted green fluorescence protein.
SEQ ID NO:7; PCR primer Us-EcoRI-1 to amplify a gene encoding red-shifted green fluorescence protein.
SEQ ID NO:8; PCR primer Us-HindIII-1 to amplify a gene encoding red-shifted green fluorescence protein.
SEQ ID NO:9; PCR primer U100HindIII-1 to amplify a portion of gene encoding red-shifted green fluorescence protein.
SEQ ID NO:10; Chimeric oligonucleotide ss Oligo. "nucleotides 1 to 4 and 50 to 53 are 2'-O-methyluridine".
SEQ ID NO:11; PCR primer RNA-ecoRI to amplify a portion of gene encoding red-shifted green fluorescence protein. nucleotides 1 to 6 are 2'-O-methylribonucleotides - other nucleotides are deoxyribonucleotides"
SEQ ID NO:12; PCR primer RNA-hindIII to amplify a portion of gene encoding red-shifted green fluorescence protein, "nucleotides 1 to 6 are 2'-O-methyl ribonucleotides - other nucleotides are deoxyribonucleotides"
SEQ ID NO:14; PCR primer GFP-κB1 to amplify a portion of gene encoding red-shifted green fluorescence protein.
SEQ ID NO:15; PCR primer GFP-κB2 to amplify a portion of gene encoding red-shifted green fluorescence protein.
SEQ ID NO:16; PCR primer GFP-κB3 to amplify a portion of gene encoding red-shifted green fluorescence protein.

## Claims

1. A method for introducing a mutation into a nucleotide sequence of a target nucleic acid, the method comprising the steps of:
(1) preparing a double-stranded DNA having an inverted repeat sequence, wherein a sense strand sequence and an antisense strand sequence are arranged in tandem and wherein the nucleotide sequence of the DNA having an inverted repeat sequence is homologous to a target nucleic acid and contains a mutation to be introduced into the target nucleic acid or a single-stranded DNA obtained by denaturing said double-stranded DNA; and
(2) transferring the single or double-stranded DNA having an inverted repeat sequence into a cell, which is not part of a human or animal body and which is not a human germ cell.

2. The method according to claim 1, wherein the DNA having an inverted repeat sequence has a binding motif sequence for a protein having a nuclear transport signal.

3. The method according to claim 2, wherein the binding motif sequence for a protein having a nuclear transport signal is a binding motif sequence for a transcription factor.

4. The method according to claim 1, wherein the DNA having an inverted repeat sequence has a modified nucleotide.

5. The method according to claim 1, wherein the target nucleic acid is a nucleic acid located in cytoplasm.

6. The method according to claim 1, wherein the target nucleic acid is a nucleic acid located in nucleus.

7. The method according to claim 1, wherein a plurality of mutations are simultaneously introduced into the target nucleic acid.

8. The method according to claim 1, wherein the mutation to be introduced into the target nucleic acid is substitution, deletion and/or insertion of a nucleotide.

9. A kit for introducing a mutation into a target nucleic acid by the method defined by claim 1, the kit containing a double-stranded DNA having an inverted repeat sequence, wherein a sense strand sequence and an antisense strand sequence are arranged in tandem, wherein the DNA having an inverted repeat sequence has a binding motif sequence for a protein having a nuclear transport signal and wherein the nucleotide sequence of the DNA having an inverted repeat sequence is homologous to a target nucleic acid and contains a mutation to be introduced into the target nucleic acid or a single-stranded DNA obtained by denaturing said double-stranded DNA.

10. The kit according to claim 9, wherein the binding motif sequence for a protein having a nuclear transport signal is a binding motif sequence for a transcription factor.

11. The kit according to claim 9, wherein the DNA having an inverted repeat sequence has a modified nucleotide.

12. Use of a double-stranded DNA having an inverted repeat sequence, wherein a sense strand sequence and an antisense strand sequence are arranged in tandem, and wherein the nucleotide sequence of the DNA having an inverted repeat sequence is homologous to a target nucleic acid and contains a mutation to be introduced into the target nucleic acid or of a single-stranded DNA obtained by denaturing said double-stranded DNA for the production of a medicament for introducing a mutation into a nucleotide sequence of a target nucleic acid in a cell, that is not a human germ line cell, for gene therapy.

## Patentansprüche

1. Verfahren zum Einführen einer Mutation in eine Nukleotidsequenz einer Zielnukleinsäure, wobei das Verfahren die Schritte umfasst:
(1) Herstellen einer doppelsträngigen DNA mit einer umgekehrt wiederholten Sequenz, wobei eine Sense-Strangsequenz und eine Antisense-Strangsequenz hintereinander angeordnet sind und wobei die Nukleotidsequenz der DNA mit einer umgekehrt wiederholten Sequenz homolog zu einer Zielnukleinsäure ist und eine Mutation enthält, die in die Zielnukleinsäure eingeführt werden soll, oder einer einzelsträngigen DNA, die durch Denaturieren der doppelsträngigen DNA erhalten wird; und
(2) Transferieren der einzelsträngigen oder doppelsträngigen DNA mit einer umgekehrt wiederholten Sequenz in eine Zelle, die nicht Teil eines menschlichen oder tierischen Körpers ist und die keine menschliche Keimzelle ist.

2. Verfahren nach Anspruch 1, wobei die DNA mit einer umgekehrt wiederholten Sequenz eine Bindungsmotivsequenz für ein Protein mit einem nukleären Transportsignal aufweist.

3. Verfahren nach Anspruch 2, wobei die Bindungsmotivsequenz für ein Protein mit einem nukleären Transportsignal eine Bindungsmotivsequenz für einen Transkriptionsfaktor ist.

4. Verfahren nach Anspruch 1, wobei die DNA mit einer umgekehrt wiederholten Sequenz ein modifiziertes Nukleotid aufweist.

5. Verfahren nach Anspruch 1, wobei die Zielnukleinsäure eine im Cytoplasma lokalisierte Nukleinsäure ist.

6. Verfahren nach Anspruch 1, wobei die Zielnukleinsäure eine im Zellkern lokalisierte Nukleinsäure ist.

7. Verfahren nach Anspruch 1, wobei eine Vielzahl von Mutationen gleichzeitig in die Zielnukleinsäure eingeführt wird.

8. Verfahren nach Anspruch 1, wobei die Mutation, die in die Zielnukleinsäure eingeführt werden soll, eine Substitution, Deletion und/oder Insertion eines Nukleotids ist.

9. Kit zum Einführen einer Mutation in eine Zielnukleinsäure durch das durch Anspruch 1 definierte Verfahren, wobei das Kit enthält: eine doppelsträngige DNA mit einer umgekehrt wiederholten Sequenz, wobei eine Sense-Strangsequenz und eine Antisense-Strangsequenz hintereinander angeordnet sind, wobei die DNA mit einer umgekehrt wiederholten Sequenz eine Bindungsmotivsequenz für ein Protein mit einem nukleären Transportsignal aufweist und wobei die Nukleotidsequenz der DNA mit einer umgekehrt wiederholten Sequenz homolog zu einer Zielnukleinsäure ist und eine Mutation enthält, die in die Zielnukleinsäure eingebracht werden soll, oder eine einzelsträngige DNA, die durch Denaturieren der doppelsträngigen DNA erhalten wird.

10. Kit nach Anspruch 9, wobei die Bindungsmotivsequenz für ein Protein mit einem nukleären Transportsignal eine Bindungsmotivsequenz für einen Transkriptionsfaktor ist.

11. Kit nach Anspruch 9, wobei die DNA mit einer umgekehrt wiederholten Sequenz ein modifiziertes Nukleotid aufweist.

12. Verwendung einer doppelsträngigen DNA mit einer umgekehrt wiederholten Sequenz, wobei eine Sense-Strangsequenz und eine Antisense-Strangsequenz hintereinander angeordnet sind und wobei die Nukleotidsequenz der DNA mit einer umgekehrt wiederholten Sequenz homolog zu einer Zielnukleinsäure ist und eine Mutation enthält, die in die Zielnukleinsäure eingeführt werden soll, oder einer einzelsträngigen DNA, die durch Denaturieren der doppelsträngigen DNA erhalten wird, zur Herstellung eines Medikaments zum Einführen einer Mutation in eine Nukleotidsequenz einer Zielnukleinsäure in einer Zelle, die keine humane Keimbahnzelle ist, zur Gentherapie.

## Revendications

1. Méthode d'introduction d'une mutation dans une séquence nucléotidique d'un acide nucléique cible, la méthode comprenant les étapes de :
(1) préparation d'un ADN double-brin ayant une séquence répétée inverse, dans laquelle une séquence de brin sens et une séquence de brin antisens sont disposées en tandem et dans laquelle la séquence nucléotidique de l'ADN ayant une séquence répétée inverse est homologue d'un acide nucléique cible et contient une mutation à introduire dans l'acide nucléique cible ou un ADN simple brin obtenu en dénaturant ledit ADN double brin ; et
(2) transfert de l'ADN simple ou double brin ayant une séquence répétée inverse dans une cellule, qui ne fait pas partie d'un corps humain ou animal et qui n'est pas une cellule germinale humaine.

2. Méthode selon la revendication 1, dans laquelle l'ADN comportant une séquence répétée inverse a une séquence d'un motif de liaison à une protéine ayant un signal de transport nucléaire.

3. Méthode selon la revendication 2, dans laquelle la séquence d'un motif de liaison à une protéine ayant un signal de transport nucléaire est une séquence d'un motif de liaison à un facteur de transcription.

4. Méthode selon la revendication 1, dans laquelle l'ADN ayant une séquence répétée inverse a un nucléotide modifié.

5. Méthode selon la revendication 1, dans laquelle l'acide nucléique cible est un acide nucléique situé dans le cytoplasme.

6. Méthode selon la revendication 1, dans laquelle l'acide nucléique cible est un acide nucléique situé dans le noyau.

7. Méthode selon la revendication 1, dans laquelle une pluralité de mutations sont simultanément introduites dans l'acide nucléique cible.

8. Méthode selon la revendication 1, dans laquelle la mutation à introduire dans l'acide nucléique cible est une substitution, délétion et/ou insertion d'un nucléotide.

9. Kit d'introduction d'une mutation dans un acide nucléique cible par la méthode définie par la revendication 1, le kit contenant un ADN double brin ayant une séquence répétée inverse, dans laquelle une séquence de brin sens et une séquence de brin antisens sont disposées en tandem, dans laquelle l'ADN ayant une séquence répétée inverse a une séquence d'un motif de liaison à une protéine ayant un signal de transport nucléaire et dans laquelle la séquence nucléotidique de l'ADN ayant une séquence répétée inverse est homologue d'un acide nucléique cible et contient une mutation à introduire dans l'acide nucléique cible ou un ADN simple brin obtenu par dénaturation dudit ADN double brin.

10. Kit selon la revendication 9, dans lequel la séquence d'un motif de liaison à une protéine ayant un signal de transport nucléaire est une séquence d'un motif de liaison à un facteur de transcription.

11. Kit selon la revendication 9, dans lequel l'ADN ayant une séquence répétée inverse a un nucléotide modifié.

12. Utilisation d'un ADN double brin ayant une séquence répétée inverse, dans lequel une séquence de brin sens et une séquence de brin antisens sont disposées en tandem, et dans lequel la séquence nucléotidique de l'ADN ayant une séquence répétée inverse est homologue d'un acide nucléique cible et contient une mutation à introduire dans l'acide nucléique cible ou d'un ADN simple brin obtenu en dénaturant ledit ADN double brin pour la production d'un médicament destiné à introduire une mutation dans une séquence nucléotidique d'un acide nucléique cible dans une cellule, qui n'est pas une cellule de lignée germinale humaine, pour la thérapie génique.
